# EUROPEAN PATENT APPLICATION

(11) **EP 3 744 331 A1**
(43) Date of publication of application: **02.12.2020**
(21) Application number: 20460023.3
(22) Date of filing: 13.05.2020
(51) Int. Cl.: A61K 31/56, A61K 47/40, A61K 47/54, A61P 25/28

(54) **COMBINATION OF BETULIN AND CYCLODEXTRIN FOR USE IN THE TREATMENT OF ALZHEIMER'S DISEASE**

(30) Priority: 31.05.2019 PL 43011619
(71) Applicant: Instytut Badawczy Innowacyjno Rozwojowy "Biotomed" Sp. Z O.O., 15-875 Bialystok (PL)
(72) Inventor: TOMULEWICZ, Mikolaj, 15-507 Bialystok (PL)

(57) **Abstract**

The present invention relates to a combination of neuroprotective pentacyclic triterpenes that are used to prevent the development and treatment of Alzheimer's disease and a pharmaceutical composition containing such a compound or compounds.
The invention relates to a combination of a pentacyclic triterpene compound represented by the formula 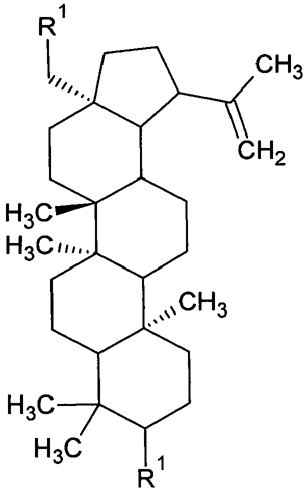 wherein the R¹ substituent is a -OH (betulin) group with cyclodextrin, for use in the prevention of Alzheimer's disease, wherein the manufactured pharmaceutical preparation contains an effective amount of the compound.

## Description

The present invention relates to a combination of neuroprotective pentacyclic triterpenes that are used to prevent the development and treatment of Alzheimer's disease and a pharmaceutical composition containing such a compound or compounds.

Triterpenes belong to the group of organic compounds. Next to sterols, saponins and cardiac glycosides, they belong to the group of triterpenoids. Pentacyclic triterpenes are characterized by the presence of five six-carbon or one five- and four six-carbon rings. Their skeleton is formed of six isoprene units and is formed as a result of cyclization of the squalene molecule. These compounds are usually colorless, crystalline, hardly reactive and have a high melting point. Many years of experience have allowed to describe over four thousand triterpenoids and their derivatives. Triterpene compounds are usually found in bark, cork, resin, skin and waxy coating of leaves and flowers, acting as a protective function against insects and microbes. They are widely distributed in the world of plants and are the subject of numerous phytochemical and pharmacological studies.
One of the natural sources of lupane triterpenes is the outer layer of the bark of white birch species (*Betula*) e.g. *B. verrucosa, B. pendula, B. pubescens, B. alba.* Birch bark is a byproduct of wood processing and is practically not used in economic activity. According to various sources, birch bark contains up to 40% of betulin and up to 5-10% of lupeol. Birch bark extracts contain betulin and oxidized derivatives: betulinic acid, betulinic aldehyde, betulinic acid methyl ester, betulaldehyde, betulonic acid, and a large number of related compounds.
Plants with a high content of pentacyclic triterpenes are used in phytotherapy because of the medicinal properties they possess.

### Antitumor activity

Triterpene compounds have anti-tumor activity at many stages of tumor formation. In preclinical studies, both in vitro and in vivo, they were shown to be active against colon, breast, prostate or melanoma cancers [Patlolla and Rao, 2012].

### Antioxidant and cytoprotective activity

Oxidative stress plays an important role in the course of cancer transformation. Increased free radical production has been found in cancer cells, which may lead to genomic instability, DNA mutation, increase in proliferation rate and potential resistance to therapy [Scibior-Bentkowska and Czeczot, 2009]. Triterpenes have cytoprotective and anti-tumor effects due to their action against free radicals. Lupeol caused a decrease in the level of lipid peroxidation, the number of free radicals and an increase in the antioxidant activity of enzymes in mice with prostate cancer [Prasad et al., 2008]. Its hepatoprotective effect has also been demonstrated.

### Effect on apoptosis

As a result of tumor transformation, the ability of cells to apoptosis disappears. Restoring the ability of apoptosis to cancer cells is a potential target for anti-cancer drugs. Triterpene compounds induce a number of mechanisms for activating apoptosis via the extrinsic and intrinsic pathways. Triterpene derivatives have been tested for their ability to induce apoptosis in cancer cells. For individual derivatives, a number of mechanisms of apoptosis activation through the extrinsic and intrinsic pathways have been proven.

### Effect on angiogenesis

An important stage of carcinogenesis is the vascularization of a growing tumor, without which the tumor remains in the pre-invasive stage. Active angiogenesis conditions further tumor cell growth and metastasis. The process of angiogenesis is induced, among others by vascular endothelial growth factor (VEGF), fibroblast growth factor (FGF), tumor growth factor (TGF) and interleukin 8 (IL-8). Some triterpene compounds inhibit the activity of the above-mentioned growth factors, thereby inhibiting angiogenesis.

### Cell protection through antioxidant activity

Free radicals, both aerobic and other, contribute to organ damage and DNA degradation, aging of the body and the formation of many diseases. Pentacyclic triterpenes have antioxidant activity, which is associated with their potential use as cytoprotective agents [Gomes Rde et al., 2009].

### Anti-inflammatory activity

Triterpenes have proven anti-inflammatory activity consisting in inhibiting the activity of enzymes participating in the inflammatory reaction, such as phospholipase A2, cyclooxygenase, lipoxygenase, nitric oxide synthase, elastase. Another mechanism that inhibits the inflammatory response of tissues is a decrease in the production of prostaglandins and pro-inflammatory cytokines: tumor necrosis factor (TNF-α), interferon (IFN-γ), interleukin (IL). Titerpenes may also reduce the activity or number of cells involved in the inflammatory process [Jiang et al., 2018].

### Antidiabetic activity

Pentacyclic triterpenes also show hypoglycemic effects in animal studies. The observed effects of lowering blood glucose, glycemic control, increasing glucose tolerance and insulin sensitivity are probably associated with stimulation of insulin synthesis and secretion by pancreatic cells [Mandal et al., 2015].

### Gastrointestinal and anti-ulcer activity

Pentacyclic triterpenes also have anti-ulcer activity. Their action is to reduce inflammatory changes in the gastric mucosa [Tamashiro Filho et al., 2012].

### Antimicrobial action

Triterpenes are also used in the prevention and therapy of diseases caused by viruses, bacteria, fungi and protozoa. They show an activity that inhibits the activity of the HIV virus [Zhu et al., 2019] as well as the Herpes simplex virus [Ikeda et al., 2005]. Antibacterial activity of pentacyclic triterpenes has been demonstrated towards among others Mycobacterium tuberculosis or Streptococcus pneumoniae [Yu et al., 2019].

Alzheimer's disease (AD) is recognized by the World Health Organization (WHO) as a global priority for public health and poses a serious challenge to the health and social care system. Despite the significant increase of knowledge about the pathogenesis of AD and how the disease has been conceptualized since Alois Alzheimer described her first case in 1907, there are still no known ways of treating or preventing it. Nowadays and with the current state of knowledge it is an incurable disease. Alzheimer's disease is the cause of dementia, accounting for 50-70% of all cases. Symptoms of Alzheimer's include thinking disorder, speech disorder, and getting lost. Dementia itself as an acquired progressive cognitive impairment sufficient to influence daily activities - is a major cause of addiction, disability and mortality. Current estimates indicate that today there are almost 44 million people with Alzheimer's disease in the world, and another new case is diagnosed every 4 seconds. According to WHO data, by 2030 this number will increase to 65 million, and in 2050 to 115 million. It is estimated that in Poland the population of people over 65 is currently about 14.7%, in 2035 it will increase to 24.5%, and in 2050 it will reach over 30%. According to data from the Central Statistical Office in Poland, there are currently over 300,000 people with Alzheimer's disease, and by 2050 this number will triple and amount to almost 1 million patients. Most cases of Alzheimer's disease appear spontaneously.

Alzheimer's disease is a chronic neurodegenerative disorder characterized by progressive loss of memory and cognitive functions. In people with developing Alzheimer's disease, increasing learning and memory impairment after all leads to the development of a full-blown disease. In a small percentage, problems with language, executive functions, perception (agnosia) or movement (apraxia) are more important than problems with memory.

The cause of most cases of Alzheimer's disease is unknown, with the exception of 1-5% of cases where the disease was genetic. β-amyloid deposition and neurofibrillary tangles, the main neuropathological features of Alzheimer's disease, occur in selectively sensitive areas of the brain, such as the hippocampus and forebrain cortex.

There are several competing hypotheses trying to explain the cause of the disease. Based on these hypotheses, so far few drugs for Alzheimer's disease have been proposed, and moreover their effectiveness is not high enough.

Among the many factors listed as responsible for Alzheimer's disease, the most important seem to be genetic factors, aggregation of β-amyloid and tau protein.

Extracellular deposits of β-amyloid are the primary cause of the disease, which leads to disturbances in interneuronal communication, resulting in the formation of neurotoxins and, consequently, leading to the death of neurons.

β-amyloid, the main component of amyloid plaques, is produced from the amyloid precursor protein (*Amyloid Precursor Protein,* APP) through a series of protease cleavages, using enzymes called secretases. As a result of this process, N-terminal, shorter fragments are formed, which perform various, important, but not yet recognized functions. The gene encoding the APP protein is mutated by the same type of enzyme that allows HIV infection.

Based on the above hypothesis, it was proposed to introduce some antiretroviral drugs for treatment. Indeed, it was found that the experimental vaccine against the fragment of APP, A_{β1-42} protein removes amyloid plaques, but did not have a significant effect on dementia [Holmes et al., 2008]. The assessment of verubecestat, which inhibits β-secretase 1, responsible for the formation of β-amyloid, was discontinued because no positive clinical effect was observed [Egan et al., 2018].

The search for drugs for the treatment of Alzheimer's disease is still a big problem because there is no specific goal to fight the disease. From many substances, natural compounds as potential drugs have also been studied. Preclinical studies have provided very promising results, matching or surpassing the potency and effect of drugs used in standard clinics.

From the Chinese patent CN106265793, the use of an extract of the mangrove plant *Acanthus ilicifolius L* is known for the preparation of drugs for the treatment and/or prevention of Alzheimer's disease.

From the international patent WO/2018/133563, plant extract of Panax and its pharmaceutical composition are known for to make a medicine for chronic heart failure, diabetes or Alzheimer's disease. On the other hand, another international patent WO/2018/096039 discloses a method for producing an extract of *Tagetes L* (marigold) enriched with leontopodic acid B and a method for producing a pharmaceutical composition serving in particular as a medicine for the prevention and/or treatment of neurodegenerative disease, in particular Alzheimer's disease.

From the European patent EP 2712619 a pharmaceutical composition is known containing extracts from the species Monsonia sp. for the prevention and/or treatment of dementia with an excellent β-amyloid inhibitory effect, which is known as an Alzheimer's causative agent.

It has been found that pentacyclic triterpene compounds have neuroprotective properties, which can be extremely useful in preventing and treating Alzheimer's disease.

The invention relates to a combination of a pentacyclic triterpene compound represented by the formula 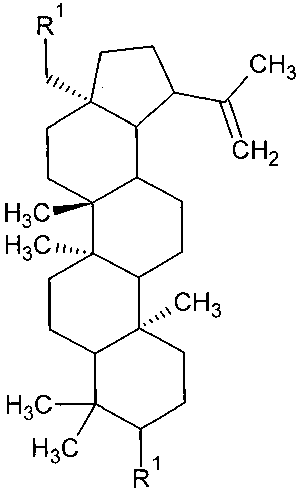 wherein the R¹ substituent is a -OH (betulin) group with cyclodextrin, for use in the prevention of Alzheimer's disease, wherein the manufactured pharmaceutical preparation contains an effective amount of the compound.
According to the invention, a combination of a pentacyclic triterpenes compound represented by the formula 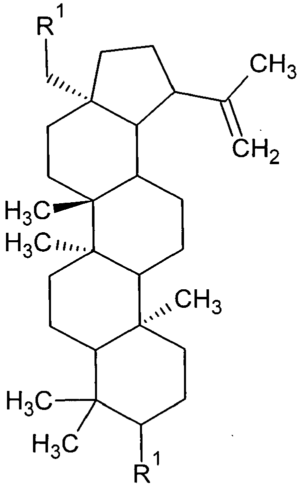 wherein the R¹ substituent is the -OH (betulin) group with cyclodextrin is used to treat Alzheimer's disease, wherein the medicament produced contains an effective amount of the compound.
The pharmaceutical composition **according to the invention** comprises combinations of a pentacyclic triterpene compound represented by the formula 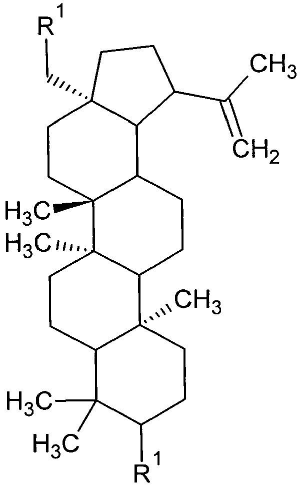 wherein R¹ is -OH (betulin) with cyclodextrin, in combination with at least one carrier selected from the following groups of compounds including **polymers:** polyvinylpyrrolidone, polyvinyl alcohol, polyethylene glycol 400, polyethylene glycol 4000; **polysaccharides:** cellulose derivatives (hydroxypropyl methylcellulose and hydroxypropyl cellulose), hypromellose phthalate, sugars and polyhydric alcohols, sorbitol, mannitol, anhydrous lactose, dextrose, ethyl cellulose, methylcellulose, cellulose, carboxymethylcellulose, starch; **gums:** acacia, tragacanth; **porous silica carriers:** aluminum silicate, calcium silicate, silicon dioxide; **salts of inorganic compounds:** calcium sulfate dihydrate, dibasic calcium phosphate, magnesium stearate.

### Investigation of betulin neuroprotective mechanisms

The study was conducted on Wistar rats weighing 220-250 g. All animals were subjected to two weeks of observation prior to the experiment to exclude infection. Rats were housed in standard cages, 5 individuals in each. The animals stayed in a room with a temperature of 22 ± 2 ° C, air humidity 60 - 70% and in natural lighting (12/12 in a day / night cycle). The animals had free access to water and standard laboratory feed *ad libitum.*

The Alzheimer's disease model was induced by administering aluminum chloride AlCl₃ (200 mg/kg body weight, i.g.) to the animals for 120 days. Aluminium is highly neurotoxic, and chronic exposure to AlCl₃ causes a significant increase in the activity of acetylcholinesterase, expression of the precursor protein amyloid, TNFα [Rodella et al., 2008; Rather et al., 2018]. Animals were assigned to groups: control group receiving saline (sham operation), group with AlCl₃ without treatment and group with AlCl₃ receiving betulin (100 mg/kg/day, i.g., within the last 50% of the days of the experiment) and betulin in the complex with cyclodextrin (100 mg/kg/day, i.g., in the last 50% of the days of the experiment). Betulin or betulin in a complex with cyclodextrin was administered orally with an intragastric probe in a 2% starch emulsion, reflecting the composition of the finished preparation given in the examples of use.
On the last day of the experiment, the animals were anaesthetized. Brains were quickly collected and then divided into different areas. The entire procedure was carried out in a cold room (+4°C) on ice, and the samples were frozen in liquid nitrogen and stored in an ultra-low temperature refrigerator for further analysis.

In order to measure rat brain damage caused by aluminum chloride, biochemical determinations were carried out on the collected brain tissue fragments.

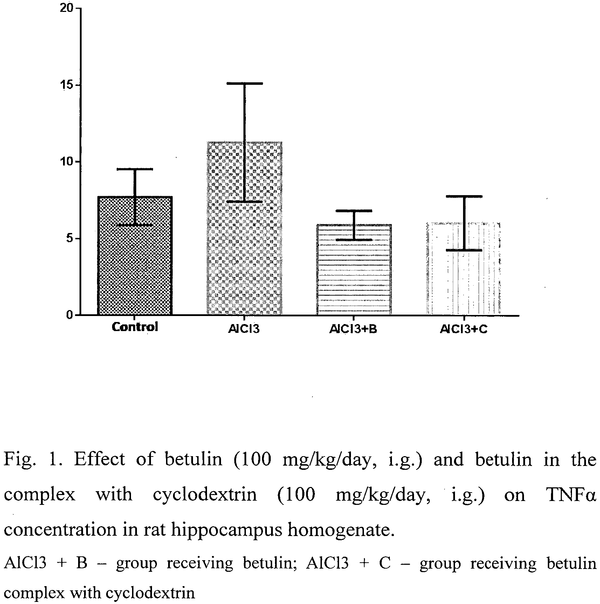

The obtained test results are given in Table 1 and in Figure 1. Neuroprotective effects were shown in all treatment groups administered betulin (100 mg/kg/day, i.g.) or betulin in the complex with cyclodextrin (100 mg/kg/day, i.g.). Betulin reduced the concentration of TNFα in the hippocampal homogenates of the brains of the animals studied. Much better effects were observed in the group given betulin in the complex with cyclodextrin.

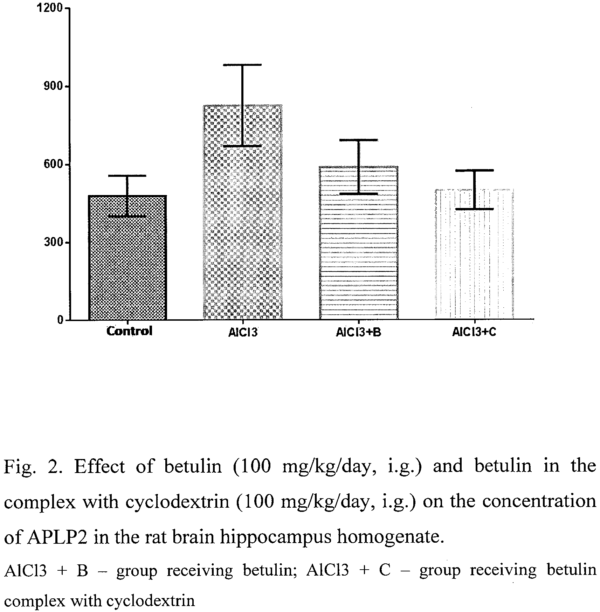

Similarly, Table 2 and Figure 2 present the results of studies on the effect of betulin on the concentration of the precursor protein APLP2. Neuroprotective effects have been demonstrated in all treatment groups given betulin (100 mg/kg/day, i.g.) or betulin in the complex with cyclodextrin (100 mg/kg/day, i.g.). Betulin reduced the concentration of APLP2 in the hippocampal homogenates of the brains of the animals studied. As in the previous case, much better effects were observed in the group to which betulin was administered in combination with cyclodextrin.

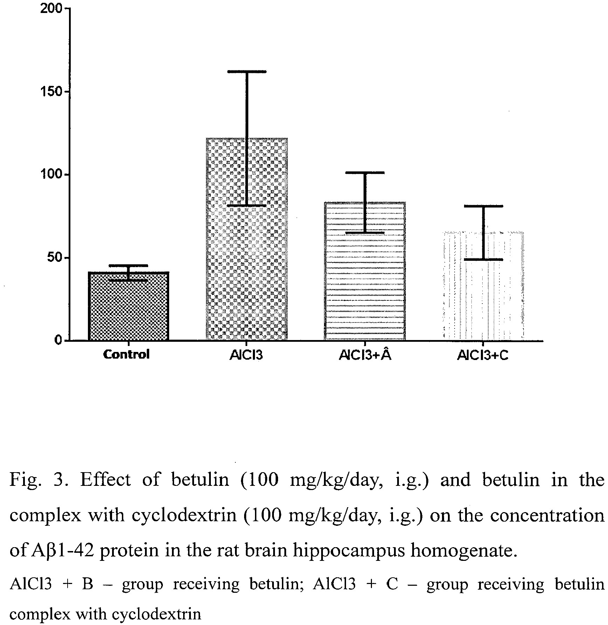

Table 3 and Figure 3 present the results of studies on the effect of betulin on the concentration of β-amyloid. Neuroprotective effects have been demonstrated in all treatment groups given betulin (100 mg/kg/day, i.g.) or betulin in the complex with cyclodextrin (100 mg/kg/day, i.g.). Betulin reduced the concentration of β-amyloid in the hippocampal homogenates of the brains of the animals studied. Also in this case, much better effects were observed in the group to which betulin was administered in a complex with cyclodextrin.

**Table 1. Effect of betulin (100 mg/kg/day, i.g.) and betulin complex with cyclodextrin (100 mg/kg/day, i.g.) on TNFα concentration in rat hippocampal homogenate.**

| Parameter | Control | AlCl3 | AlCl3+B | AlCl3+C |
|---|---|---|---|---|
| TNF-α, pg/mg | 7.70±0.65 | 11.30±1.46^{a} | 5.88±0.39^{b} | 6.03±0.67^{b} |

| | | | | |
|---|---|---|---|---|
| - p<0.05 compared to the control group, ^{b} - p<0.05 compared to the AlCl3 group AlCl3 + B - group receiving betulin; AlCl3 + C - group receiving betulin complex with cyclodextrin | | | | |

**Table 2. Effect of betulin (100 mg/kg/day, i.g.) and betulin in the complex with cyclodextrin (100 mg/kg/day, i.g.) on the concentration of APLP2 in the rat brain hippocampus homogenate**

| Parameter | Control | AlCl3 | AlCl3+B | AlCl3+C |
|---|---|---|---|---|
| APLP2, pg/100 mg | 477,9±24,9 | 824,8±55,1^{a} | 588,1±36,4^{ab} | 499,2±26,3^{b} |

| | | | | |
|---|---|---|---|---|
| ^{a} - p<0.05 compared to the control group, ^{b} - p<0.05 compared to the AlCl3 group AlCl3 + B - group receiving betulin; AlCl3 + C - group receiving betulin complex with cyclodextrin | | | | |

**Table 3. Effect of betulin (100 mg/kg/day, i.g.) and betulin in the complex with cyclodextrin (100 mg/kg/day, i.g.) on the concentration of Aβ1-42 protein in rat hippocampal homogenate**

| Parameter | Control | AlCl3 | AlCl3+B | AlCl3+C |
|---|---|---|---|---|
| Protein Aβ₁₋₄₂, pg/100 mg | 40,92±1,56 | 121,90±14,30 ^{a} | 83,25±6,40 ^{ab} | 65,34±5,70 ^{b} |

| | | | | |
|---|---|---|---|---|
| ^{a} - p<0.05 compared to the control group, ^{b} - p<0.05 compared to the AlCl3 group AlCl3 + B - group receiving betulin; AlCl3 + C - group receiving betulin complex with cyclodextrin | | | | |

Pharmaceutical compositions with neuroprotective effect used in the prevention and treatment of Alzheimer's disease are prepared and administered in various forms. It is obvious to those skilled in the art that unit doses may contain as active ingredient one or more of the compounds described. It is also evident that both dosages and methods of administration will vary depending on the needs of the patient and depending on the particular activity of the active ingredient (s). Determining the dosage and method of administration remains within the skill of each practicing physician.

## Claims

1. A compound of the pentacyclic triterpenes compound represented by the formula 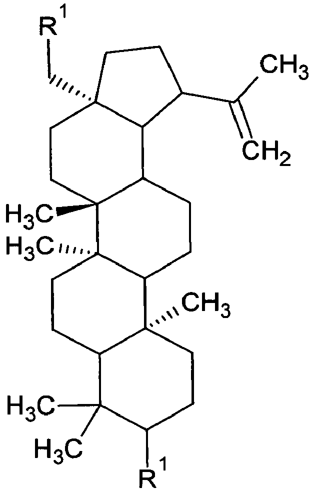 wherein the R¹ substituent is a -OH (betulin) group with cyclodextrin, for use in the prevention of Alzheimer's disease, wherein the manufactured pharmaceutical preparation contains an effective amount of the compound.

2. A compound of the pentacyclic triterpenes compound represented by the formula 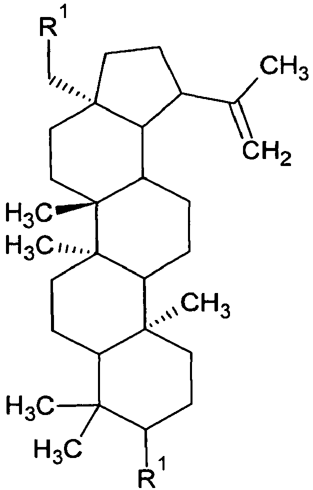 wherein the R¹ substituent is a -OH (betulin) group with cyclodextrin, for use in the treatment of Alzheimer's disease, wherein the manufactured pharmaceutical preparation contains an effective amount of the compound.

3. A pharmaceutical composition **characterized in that** it comprises combinations of a pentacyclic triterpene compound of the formula 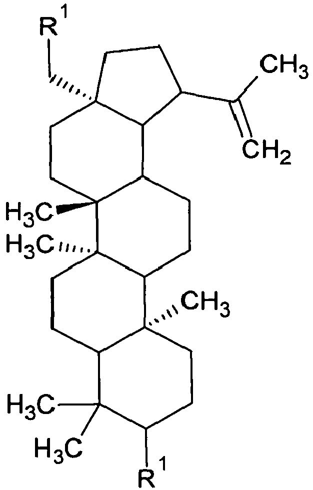 wherein R¹ is -OH (betulin) with cyclodextrin, in combination with at least one carrier selected from the following groups of compounds including **polymers:** polyvinylpyrrolidone, polyvinyl alcohol, polyethylene glycol 400, polyethylene glycol 4000; **polysaccharides:** cellulose derivatives (hydroxypropyl methylcellulose and hydroxypropyl cellulose), hypromellose phthalate, sugars and polyhydric alcohols, sorbitol, mannitol, anhydrous lactose, dextrose, ethyl cellulose, methylcellulose, cellulose, carboxymethylcellulose, starch; gums: acacia, tragacanth; **porous silica carriers:** aluminum silicate, calcium silicate, silicon dioxide; **salts of inorganic compounds:** calcium sulfate dihydrate, dibasic calcium phosphate, magnesium stearate.
